# EUROPÄISCHE PATENTSCHRIFT

(11) **EP 2 299 920 B1**
(45) Veröffentlichungstag und Bekanntmachung des Hinweises auf die Patenterteilung: **21.01.2015**
(21) Anmeldenummer: 09761610.6
(22) Anmeldetag: 27.05.2009
(51) Int. Cl.: A61B 17/80

(54) **VORRICHTUNG ZUR OSTEOSYNTHESE SOWIE ZUR FIXIERUNG UND STABILISIERUNG VON RÖHRENKNOCHEN**
DEVICE FOR OSTEOSYNTHESIS AND FOR FIXING AND STABILIZING LONG BONES
DISPOSITIF D'OSTÉOSYNTHÈSE, DE FIXATION ET DE STABILISATION D'OS LONGS

(30) Priorität: 12.06.2008 DE 102008002389
(43) Veröffentlichungstag der Anmeldung: 30.03.2011
(73) Patentinhaber: Medxpert Gmbh, 79423 Heitersheim (DE)
(72) Erfinder: REISBERG, Erhard, 79427 Eschbach (DE)
(74) Vertreter: Lermer, Christoph
(86) Internationale Anmeldenummer: PCT/EP2009/056425
(87) Internationale Veröffentlichungsnummer: WO 2009/150047

(56) Entgegenhaltungen:
- EP-A- 0 024 635
- DE-A1- 2 736 972
- DE-A1- 3 808 937
- DE-A1-102006 042 277
- FR-A- 2 353 274
- JP-A- 3 012 145
- US-A1- 2008 082 101

## Beschreibung

### GEBIET DER ERFINDUNG

Die Erfindung betrifft eine Vorrichtung zur Osteosynthese sowie zur Fixierung und Stabilisierung von Röhrenknochen, umfassend einen sich in eine erste longitudinale Richtung erstreckenden Trägersteg, wenigstens eine sich lateral vom Trägersteg erstreckende erste Klammer und eine sich lateral vom Trägersteg erstreckende zweite Klammer, wobei die zweite Klammer longitudinal versetzt von der ersten Klammer angeordnet ist.

### STAND DER TECHNIK

Nach Frakturen oder Osteotomien von Röhrenknochen, beispielsweise im Thoraxbereich, besteht die Notwendigkeit, den Brustkorb zu stabilisieren, um ein Zusammenwachsen der Knochen zu ermöglichen. Die Stabilisierung erfolgt dabei meist konservativ, also nicht invasiv, von Außen. Die Belastung für den Patienten ist dennoch erheblich. So können seine Atmung und Mobilität eingeschränkt sein. Eine Besserung des Zustands tritt erst nach einer relativ langen Dauer der Rekonvaleszenz ein.

Sofern eine Behandlung mittels eines operativen Eingriffs erfolgt, werden zur Fixierung der Knochen in der Praxis chirurgischer Draht oder Osteosynthese-Draht oder andere Materialien verwendet. Diese Methoden weisen jedoch insbesondere den Nachteil auf, dass die angestrebte Übungsstabilität, d. h. ein Zustand, bei dem eine leichte Belastung des Brustkorbs möglich ist, in der Regel nicht sofort erreicht wird.

Um dem entgegenzuwirken, wurde in der Druckschrift DE 10 2006 042 277 A1 ein Implantat zur Osteosynthese vorgeschlagen, das im Wesentlichen aus zwei Rippenklammern und einem Verbindungsstab besteht. Für die Verbindung zweier Knochenenden wird je eine Klammer an einem Knochenende fixiert, anschließend die Klammern mit dem Verbindungsstab verbunden. Die Verbindungsstäbe werden in unterschiedlichen Längen zusammen mit den Klammem in einem Set bereitgestellt. Mit Hilfe dieses Systems wird bei Frakturen oder Osteotomien die notwendige Übungsstabilität unmittelbar beim chirurgischen Eingriff hergestellt.

Das Dokument DE 27 36 972 A1 beschreibt eine Rippenplatte aus Federstahl, die über seitlich wegstehende Krallen an den Rippen befestigt werden kann. Die Krallen sind weiterhin über einen Steg untereinander verbunden, wobei der Steg in Richtung der Krallen gewölbt ist.

Das Dokument DE 38 08 937 A1 zeigt ein Implantat zur Stabilisierung von Knochenfrakturen. Das Implantat weist einen Steg auf, an dem zu beiden Seiten kammförmig angeordnete Klammern angeordnet sind.

Das Dokument EP 0 024 635 A beschreibt ein Implantat zur Befestigung von Knochen, wobei das Implantat Befestigungsklammern aufweist. Zur Befestigung werden die Klammern in den Knochen eingedrückt.

Das Dokument FR 2 353 274 A offenbart ein Implantat zur Fixierung von gebrochenen Knochen, das einen Steg umfasst, der an beiden Enden Klammern aufweist.

Im Dokument JP 03 012145 A ist ein weiteres Implantat zur Osteosynthese offenbart, wobei das Implantat einen Steg mit daran angeordneten Klammern aufweist.

Die US 2008/0082101 A1 offenbart ein Implantat bestehend aus zwei Implantatkomponenten und einem Verbindungsstab, wobei der Verbindungsstab mit Verbindungsabschnitten der Implantatkomponenten verbunden wird.

### AUFGABE DER ERFINDUNG

Ausgehend vom bekannten Stand der Technik besteht die Aufgabe der vorliegenden Erfindung darin, eine Vorrichtung zur Osteosynthese bereitzustellen, die, bei einfacher Handhabung, flexibel einsetzbar ist.

### TECHNISCHE LÖSUNG

Diese Aufgabe wird gelöst durch eine Vorrichtung zur Osteosynthese sowie zur Fixierung und Stabilisierung von Röhrenknochen gemäß dem Anspruch 1. Vorteilhafte Ausführungsformen sind Gegenstand der abhängigen Ansprüche.

Die erfindungsgemäße Vorrichtung zur Osteosynthese sowie zur Fixierung und Stabilisierung von Röhrenknochen umfasst einen sich in eine erste longitudinale Richtung erstreckenden Trägersteg, wenigstens eine sich lateral vom Trägersteg erstreckende erste Klammer und eine sich lateral vom Trägersteg erstreckende zweite Klammer, wobei die zweite Klammer longitudinal versetzt von der ersten Klammer angeordnet ist. Der Steg weist wenigstens einen zwischen der ersten Klammer und der zweiten Klammer angeordneten Bereich auf, der relativ zur longitudinalen Achse des Stegs verbiegbar und zur Anpassung an die individuellen Knochenkonturen des Patienten verformbar ausgebildet ist. Die Vorrichtung ist aus Titan hergestellt. Der Steg ist wenigstens in zwei Dimensionen relativ zur longitudinalen Achse verbiegbar sowie verformbar und um die longitudinale Achse verdrehbar sowie verformbar ausgebildet. Der Steg und die sich vom Steg erstreckenden Klammern sind einstückig ausgebildet. Jeweils eine Klammer und ein Stegbereich bilden ein Strukturelement, wo bei sich die Anordnung der Strukturelemente entlang der longitudinalen Achse des Stegs wiederholt.

Die Vorrichtung ist demnach als Implantat bzw. Rippenklammer ausgebildet, das im Wesentlichen aus wenigstens zwei sich beidseitig von einem Steg erstreckenden Klammern zur Befestigung an jeweils einem Ende eines Röhrenknochens besteht. Die erste Klammer wird zur Befestigung an einer Seite der Fraktur/Osteotomie benötigt, die zweite Klammer zur Befestigung des Implantats an der zweiten Seite der Fraktur/Osteotomie. Die Fraktur wird durch den longitudinalen Steg überbrückt.

Das Implantat ist vorzugsweise mit einer Vielzahl von Klammerpaaren ausgebildet. Insbesondere bei schrägen Frakturen/ Osteotomien kann es vorteilhaft sein, zwei bis drei Klammerpaare pro Seite zur Befestigung des Implantats zu verwenden.

Die Vorrichtung kann relativ einfach hergestellt und mit großer Steglänge bzw. einer Vielzahl von Klammerpaaren, die sich vom Steg erstrecken, bereitgestellt werden. Das Implantat kann theoretisch als "Endlosklammer" zur individuellen Kürzung durch den Chirurgen bereitgestellt werden.

Erfindungsgemäß ist der Steg zwischen den wenigstens zwei Klammern verbiegbar. Sofern ein Steg mit mehreren Klammern versehen ist, ist er vorzugsweise zwischen allen Klammern relativ zur longitudinalen Achse verbiegbar ausgebildet. Auf diese Weise kann eine Anpassung der Form des Implantats an praktisch jede Art von Knochenoberfläche bzw. -kontur vorgenommen werden.

Auch die erste Klammer und die zweite Klammer sind verbiegbar ausgebildet, um nach dem gegenseitigen In-Eingriff-Bringen mit dem Röhrenknochen an diesem fixiert zu werden.

Das Implantat kann universell an runden oder ovalen Röhrenknochen, beispielsweise an Rippenknochen, angebracht werden. Dazu wird die Klammer beidseitig der Fraktur fixiert. Die Knochensegmente werden vorher zueinander in eine optimale Lage gebracht und mittels des erfindungsgemäßen Implantats stabilisiert. Dadurch wird der biologische Prozess der Osteosynthese unterstützt. Das Implantat kann entweder nach erfolgter knöchener Durchbauung des Knochens entfernt oder im Körper belassen werden.

Die Vorrichtung kann somit in einem Set mit nur wenigen Bestandteilen und flexiblen Einsatzmöglichkeiten bereitgestellt werden. Die Handhabung durch den Chirurgen ist einfach, da zur Verbindung zweier Röhrenknochen-Enden der Einsatz lediglich eines einstücken Implantats erforderlich ist.

Insbesondere ist der Steg wenigstens in zwei Dimensionen relativ zur longitudinalen Achse verbiegbar ausgebildet. Beispielsweise kann der Steg in zwei beliebigen nicht-parallelen Ebenen, die sich in der longitudinalen Achse schneiden, gebogen werden.

Vorzugsweise kann der Steg wenigstens einen zwischen der ersten Klammer und der zweiten Klammer angeordneten Bereich aufweisen, der ein Verdrehen der ersten Klammer relativ zur zweiten Klammer um die longitudinale Achse des Stegs ermöglicht. In anderen Worten kann der Steg verbogen und/oder torquiert werden, so dass eine dreidimensionale Anpassung der 3D-Osteosynthese-Vorrichtung an jede Kontur bzw. Knochenstruktur möglich ist. Die Anpassung erfolgt beispielsweise mittels Flachzangen zum Auf- und Abbiegen des Stegs bzw. zum Torquieren. Mittels einer Dreipunkt-Biegezange kann der Steg seitlich, d. h. parallel zur Vorderseite des Stegs, relativ zur Längsachse gebogen werden. Die Dreipunkt-Biegezange ermöglicht beispielsweise ein Verbiegen um einen Winkel von ca. 15 ° zu jeder Seite hin und zwischen jedem Klammerpaar.

Zum Fixieren der ersten Klammer und der zweiten Klammer sind nach dem gegenseitigen InEingriff-Bringen mit dem Röhrenknochen kann eine Klammer-Fixierzange verwendet werden, die beispielsweise senkrecht zur Klammer eingesetzt werden kann. Es kann jedoch auch eine gewinkelte Zange verwendet werden, die den Zugang zum Implantat durch einen vorab präparierten Weichteiltunnel ermöglicht.

Des Weiteren werden möglicherweise eine Steg-Schneidezange zum Abschneiden eines Implantats geeigneter Länge und eine Dreipunkt-Biegezange verwendet, um den Steg in geeigneter Weise zu biegen.

In einer bevorzugten Ausführungsform der Erfindung erstrecken sich wenigstens die erste Klammer und/oder die zweite Klammer beidseitig vom Steg. Jede Klammer weist somit zwei Schenkel auf, die zur Befestigung an einem Röhrenknochen angeformt werden können.

Insbesondere sind die erste Klammer und/oder die zweite Klammer jeweils kreissegmentartig mit einer Eingriffsöffnung zum gegenseitigen Eingriff mit einem Röhrenknochen ausgebildet. Zur Befestigung muss der Knochen wenigstens teilweise von der Klammer hintergriffen werden. Daher können für verschiedene Anwendungen und Knochendurchmesser unterschiedlich dimensionierte Klammern zur Verfügung gestellt werden. Die Klammern werden mit einer definierten Breite, d. h. einen definierten Abstand zwischen den vorzugsweise halbkreisförmig vorgebogenen seitlichen Klammerpaaren, bereitgestellt. Die Eingriffsöffnung kann vor dem Einsetzen des Implantats beispielsweise mittels einer Flachzange weiter aufgebogen oder verkleinert werden. Zudem ist es möglich, die Vorrichtung in verschiedenen Größenvarianten für unterschiedliche Knochen bereitzustellen.

Vorzugsweise ist der Steg als Materialstreifen mit einer vorgegebenen Länge und einer Breite ausgebildet. Die Breite des Stegs kann entlang der Längsachse konstant sein, insbesondere aber auch variieren. So kann der Steg insbesondere im Bereich zwischen den Klammern, in denen der Steg verbiegbar ist, mit verringerter Breite bereitgestellt werden, um das Verbiegen, beispielsweise das seitliche Verbiegen, zu erleichtern. Die Stegbereite kann, je nach Anwendung, beispielsweise ca. 2 bis 6 mm bei einer Materialstärke von beispielsweise 1 bis 4 mm betragen.

In einer besonders bevorzugten Ausführungsform der Erfindung weist der Steg wenigstens zwei Öffnungen auf, die entlang der Längsachse des Stegs angeordnet sind. Die Öffnungen bzw. Bohrungen können entlang des zentralen Stegs in regelmäßigen Abständen ausgebildet sein.

Insbesondere weist der Steg Öffnungen zum Eingriff eines Werkzeugs zum Verbiegen und/oder Torquieren des verbiegbaren Bereichs des Stegs auf. Die Öffnungen können zum Eingriff einer Dreipunkt-Zange zum seitlichen Verbiegen des Stegs ausgebildet sein.

Insbesondere sind die Öffnungen entlang der Längsachse des Stegs auf Höhe jeweils einer Klammer angeordnet.

In einer bevorzugten Ausführungsform sind die Öffnungen zwischen den verbiegbar ausgebildeten Bereichen des Stegs angeordnet. Die Öffnungen sind im Rahmen der Erfindung derart ausgebildet und angeordnet, dass beim Eingriff eines Werkzeugs, beispielsweise einer Dreipunkt-Biegezange, beim bestimmungsgemäßen Verbiegen eines verbiegbaren Bereichs möglichst keine Verformung der Öffnung auftritt, sondern lediglich eine Verformung der verbiegbaren Bereiche des Stegs.

Insbesondere ist auf der Höhe jeder Klammer der Vorrichtung eine Öffnung vorgesehen.

Vorzugsweise sind der Steg und die sich vom Steg erstreckenden Klammern einstückig ausgebildet. Dadurch werden die Bereitstellung und die Handhabung des Implantats erheblich erleichtert, da zum Einen eine Standardklammer für verschiedenste Frakturen zur Verfügung gestellt werden und vom Chirurgen auf die gewünschte Länge verkürzt werden kann, zum Anderen der Chirurg keine Einzelteile während der Operation zusammenfügen muss.

Vorzugsweise ist die Vorrichtung aus einem implantationsfähigen Material, insbesondere aus Titan, hergestellt. Die Vorrichtung kann jedoch aus allen möglichen für die Implantation in einen menschlichen Körper geeigneten Materialien, wie aus geeigneten Metallen, resorbierbaren Materialen, Memory-Werkstoffen, etc., hergestellt sein.

Rein-Titan ist wegen seiner Biokompatibilität und Bioinertheit besonders geeignet. Titan ist somit auch als Langzeitimplantat geeignet, während herkömmlicher Implantat-Stahl seine Körperverträglichkeit mit der Zeit verliert. Da sich die Oxidschicht des Titans auch nach dem Verbiegen im biologischen Milieu spontan erneuert, bleiben Titan-Implantate auch über lange Zeit chemisch inert und korrosionsbeständig. Im Hinblick auf die vorliegende Erfindung sind Titan-Implantate relativ exakt verformbar und lassen sich somit genau an die individuellen Knochenkonturen des Patienten anpassen.

Vorzugsweise weist die Vorrichtung eine Vielzahl von Klammern und/oder eine Vielzahl von Öffnungen auf, wobei sich die Struktur der Vorrichtung entlang der Längsachse in regelmäßigen Abständen wiederholt. Es werden zwei oder mehrere Klammern benötigt, die longitudinal versetzt am Steg angeordnet sind. Vorzugsweise weist die Vorrichtung mehr als zwei Klammern auf, die entlang der longitudinalen Achse des Stegs in regelmäßigen Abständen angeordnet sind. Auf Höhe jeder Klammer kann eine Öffnung zum Eingriff eines Biegewerkzeugs angeordnet sein.

In einer besonders bevorzugten Ausführungsform der Erfindung ist die Vorrichtung aus Strukturelementen, umfassend jeweils eine Klammer und einen Stegbereich, aufgebaut, wobei sich die Struktur n-mal wiederholt, mit n = 1, 2, 3, ... Somit wird ein einfaches Implantat mit einer insgesamt regelmäßigen Struktur bereitgestellt, das mit Hilfe einer Schneidezange auf eine gewünschte Länge abgeschnitten werden kann, sobald diese feststeht.

### KURZE BESCHREIBUNG DER FIGUREN

Weitere Merkmale und Vorteile der Erfindung ergeben sich aus der Beschreibung eines bevorzugten Ausführungsbeispiels anhand der beigefügten Zeichnungen. Es zeigen:
- Figur 1: eine perspektivische Ansicht eines erfindungsgemäßen Implantats;
- Figur 2: eine Draufsicht auf das erfindungsgemäße Implantat; und
- Figur 3: eine Seitenansicht des erfindungsgemäßen Implantats.

### BESCHREIBUNG EINES BEVORZUGTEN AUSFÜHRUNGSBEISPIELS

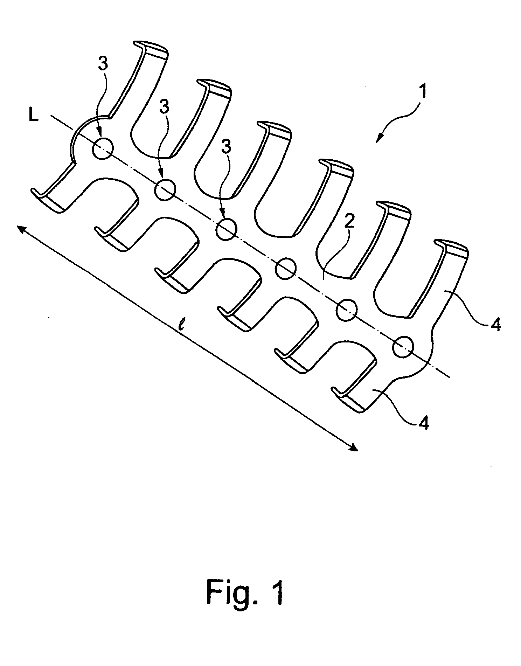

In der Figur 1 ist ein erfindungsgemäßes Implantat 1 dargestellt. Dieses weist einen zentralen Steg 2 auf, der sich entlang einer Längsachse L erstreckt. Der Steg 2 weist einen Länge 1 auf, in der in regelmäßigen Abständen Öffnungen 3 ausgebildet sind. Auf Höhe der Öffnungen 3 erstrecken sich beidseitig im rechten Winkel jeweils Klammern (Klammerpaare) 4, die beim Einsetzen des Implantats 1 in gegenseitigen Eingriff mit einem Röhrenknochen gebracht und an diesem festgeklemmt werden.

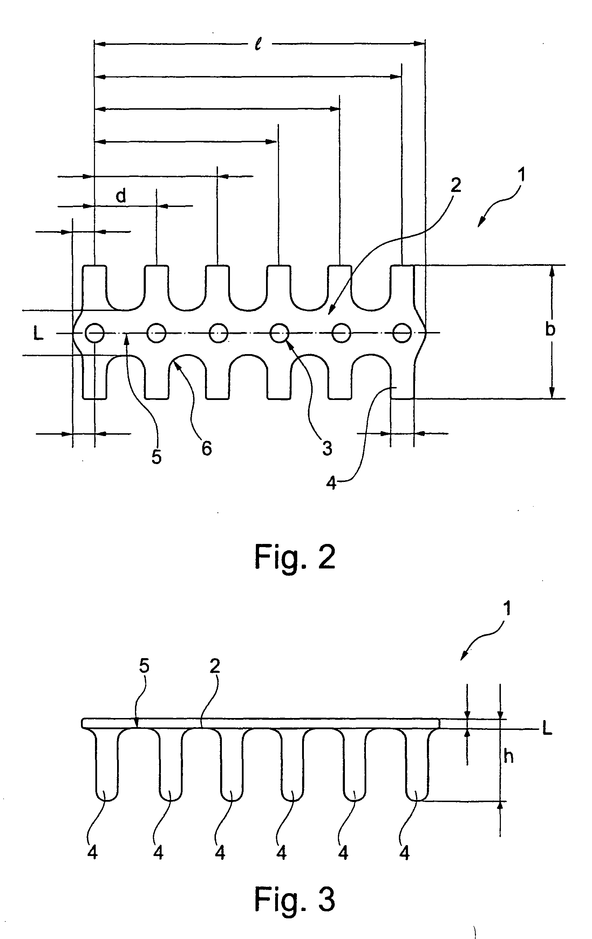

Die Figur 2 zeigt schematisch das Implantat 1 in einer Draufsicht. Die Öffnungen 3 und die Klammern 4 sind in regelmäßigen Abständen d am Steg 2 angeordnet bzw. ausgebildet. Die Breite b des Implantats 1 wird durch den Abstand der halbkreisförmig sich in die Bildebene erstreckenden Klammern 4 definiert.

Der Steg 2 ist als dünner Materialstreifen ausgebildet, in dem die Öffnungen 3 zum Eingriff eines Biegewerkzeugs, insbesondere einer Dreipunkt-Zange, ausgebildet sind. Das Verbiegen erfolgt einerseits in der Zeichenebene relativ zur Längsachse L, insbesondere in den Bereichen 5 zwischen den Öffnungen 3. In diesen Bereichen 5 weist der Steg 2 radiale Konturen 6 auf, so dass - in der Bildebene - Verengungen entstehen, um das Verbiegen und Verdrehen in den Bereichen 5 zu erleichtern.

Neben dem Verbiegen in der Zeichenebene kann der Steg 2 in den Bereichen 5 senkrecht oder schräg zur Zeichenebene relativ zur Längsachse L gebogen werden. Diese Verbiegung kann beispielsweise mit Hilfe einer Flachzange durchgeführt werden. Darüber hinaus kann der Steg 2 durch Ausüben eines Drehmoments um die Längsachse L verdreht (torquiert) werden. So werden zwei benachbarte Klammern 4 relativ zueinander verdreht. Insgesamt ist auf diese Weise eine dreidimensionale Anpassung des Implantats 1 an eine gegebene Knochenstruktur möglich.

In der Figur 3 ist das Implantat 1 in einer Seitenansicht dargestellt. Vom als dünnen Streifen ausgebildeten Steg 2 erstrecken sich seitlich nach unten die Klammern 4. Die Höhe h des Implantats 1 wird durch den Abstand der Oberseite des Stegs 2 bis zu den unteren Enden der Klammern 4 bestimmt. Der Steg 2 kann insbesondere in den Bereichen 5 in der Zeichenebene relativ zur Längsachse L, senkrecht zur Zeichenebene relativ zur Längsachse L und durch Verdrehen des Stegs 2 um die Längsachse L in drei Dimensionen an eine Knochenstruktur angepasst werden.

## Patentansprüche

1. Vorrichtung (1) zur Osteosynthese sowie zur Fixierung und Stabilisierung von Röhrenknochen, umfassend einen sich in eine erste longitudinale Richtung (L) erstreckenden Trägersteg (2), wenigstens eine sich lateral vom Trägersteg (2) erstreckende erste Klammer (4) und eine sich lateral vom Trägersteg (2) erstreckende zweite Klammer (4), wobei die zweite Klammer (4) longitudinal versetzt relativ zur ersten Klammer (4) angeordnet ist, wobei jeweils eine Klammer und ein Stegbereich ein Strukturelement bilden, wobei
die Vorrichtung aus Titan hergestellt ist, und der Steg (2) wenigstens einen zwischen der ersten Klammer (4) und der zweiten Klammer (4) angeordneten Bereich (5) aufweist, der relativ zur longitudinalen Achse des Stegs (2) verbiegbar und zur Anpassung an die individuellen Knochenkonturen des Patienten verformbar ausgebildet ist, und wobei
der Steg (2) wenigstens in zwei Dimensionen relativ zur longitudinalen Achse (L) verbiegbar sowie verformbar, und um die longitudinale Achse (L) verdrehbar sowie verformbar ausgebildet ist, **dadurch gekennzeichnet, dass**
der Steg (2) und die sich vom Steg (2) erstreckenden Klammern (4) einstückig ausgebildet sind, und
sich die Anordnung der Strukturelemente entlang der Längsachse wiederholt.

2. Vorrichtung (1) nach einem der vorhergehenden Ansprüche, **dadurch gekennzeichnet, dass**
sich wenigstens die erste Klammer (4) und/oder die zweite Klammer (4) beidseitig vom Steg erstrecken.

3. Vorrichtung (1) nach einem der vorhergehenden Ansprüche, **dadurch gekennzeichnet, dass**
wenigstens die erste Klammer (4) und/oder die zweite Klammer (4) kreissegmentartig mit einer Eingriffsöffnung zum gegenseitigen Eingriff mit einem Röhrenknochen ausgebildet sind.

4. Vorrichtung (1) nach einem der vorhergehenden Ansprüche, **dadurch gekennzeichnet, dass**
der Steg (2) wenigstens zwei Öffnungen (3) aufweist, die entlang der Längsachse (L) des Stegs (2) angeordnet sind.

5. Vorrichtung (1) nach einem der vorhergehenden Ansprüche, **dadurch gekennzeichnet, dass**
der Steg (2) Öffnungen (3) zum Eingriff eines Werkzeugs zum Verbiegen und/oder Torquieren des verbiegbaren Bereichs (5) des Stegs (2) aufweist.

6. Vorrichtung (1) nach einem der Ansprüche 4 oder 5, **dadurch gekennzeichnet, dass** die Öffnungen (3) entlang der Längsachse (L) des Stegs (2) auf Höhe jeweils einer Klammer (4) angeordnet sind.

7. Vorrichtung (1) nach einem der Ansprüche 4 bis 6, **dadurch gekennzeichnet, dass** die Öffnungen (3) zwischen den verbiegbar ausgebildeten Bereichen (5) des Stegs (2) angeordnet sind.

8. Vorrichtung (1) nach einem der vorhergehenden Ansprüche, **dadurch gekennzeichnet, dass**
auf der Höhe jeder Klammer (4) der Vorrichtung (1) eine Öffnung (3) vorgesehen ist.

9. Vorrichtung (1) nach einem der vorhergehenden Ansprüche, **dadurch gekennzeichnet, dass**
die Vorrichtung (1) eine Vielzahl von Klammern (4) und/oder eine Vielzahl von Öffnungen (3) aufweist, wobei sich die Struktur der Vorrichtung (1) entlang der Längsachse (L) des Stegs (2) in regelmäßigen Abständen wiederholt.

## Claims

1. A device (1) for the osteosynthesis as well as the immobilization and stabilisation of tubular bones, comprising a carrier fillet (2) extending in a first longitudinal direction (L), at least one first clamp (4) extending laterally from the carrier fillet (2) and a second clamp (4) extending laterally from the carrier fillet (2), the second clamp (4) being longitudinally offset from the first clamp (4), wherein each respective clamp and a fillet portion form a structural element, wherein
the device is made of titanium, and the fillet (2) has at least one area (5) arranged between the first clamp (4) and second clamp (4) that is formed so as to be bendable relative to the longitudinal axis of the fillet (2) and deformable to be adapted to the individual bone structure of a patient, and wherein
the fillet (2) is bendable and deformable in at least two dimensions relative to the longitudinal axis (L), and it is twistable and deformable about the longitudinal axis (L), **characterised by** the fact that
the fillet (2) and the clamps (4) extending from the fillet (2) are formed in one piece, and
the arrangement of the structural elements repeats itself along the longitudinal axis.

2. The device (1) in accordance with claim 1,
**characterised by** the fact that
at least the first clamp (4) and/or the second clamp (4) extend from both sides of the fillet.

3. The device (1) in accordance with any of the previous claims,
**characterised by** the fact that
at least the first clamp (4) and/or the second clamp (4) is formed as a circular segment with an engagement opening for mutual engagement with a tubular bone.

4. The device (1) in accordance with any of the previous claims,
**characterised by** the fact that
the fillet (2) has at least two openings (3) that are arranged along the longitudinal axis (L) of the fillet (2).

5. The device (1) in accordance with any of the previous claims,
**characterised by** the fact that
the fillet (2) has openings (3) for engagement of a tool for bending and/or torquing the bendable area (5) of the fillet (2).

6. The device (1) in accordance with claim 4 or 5,
**characterised by** the fact that
the openings (3) along the longitudinal axis (L) of the fillet (2) are each arranged at the level of a clamp (4).

7. The device (1) in accordance with any of claims 4 to 6,
**characterised by** the fact that
the openings (3) are arranged between the bendable areas (5) of the fillet (2).

8. The device (1) in accordance with any of the previous claims,
**characterised by** the fact that
a respective opening (3) is provided at the level of each clamp (4) of the device (1).

9. The device (1) in accordance with any of the previous claims,
**characterised by** the fact that
the device (1) has a plurality of clamps (4) and/or a plurality of openings (3), with the structure of the device (1) repeated at regular intervals along the longitudinal axis (L) of the fillet (2).

## Revendications

1. Dispositif (1) d'ostéosynthèse ainsi que de fixation et de stabilisation d'os longs comprenant une traverse de support (2) qui s'étend dans une première direction longitudinale (L), au moins une première griffe (4) qui s'étend latéralement à partir de la traverse de support (2) et une seconde griffe qui s'étend latéralement à partir de la traverse de support, la seconde griffe (4) étant placée en étant décalée longitudinalement par rapport à la première griffe (4), cependant qu'une griffe et une zone d'traverse forment respectivement un élément de structure, cependant que le dispositif est fabriqué en titane et la traverse (2) présente au moins une zone (5), placée entre la première griffe (4) et la seconde griffe (4), qui est configurée flexible par rapport à l'axe longitudinal de la traverse (2) et déformable pour s'adapter aux contours individuels de l'os du patient et cependant que la traverse (2) est configurée flexible ainsi que déformable au moins dans deux dimensions par rapport à l'axe longitudinal (L) et rotatif ainsi que déformable autour de l'axe longitudinal (L), **caractérisé en ce que** la traverse (2) et les griffes (4) qui s'étendent à partir de la traverse (2) sont configurées en une pièce et que l'arrangement des éléments de structure se répète le long de l'axe longitudinal.

2. Dispositif (1) selon l'une des revendications précédentes, **caractérisé en ce que** la première griffe (4) et/ou la seconde griffe (4) s'étendent des deux côtés de la traverse.

3. Dispositif (1) selon l'une des revendications précédentes, **caractérisé en ce qu'**au moins la première griffe (4) et/ou la seconde griffe (4) sont configurées de type segment de cercle avec une ouverture d'engrènement pour l'engrènement mutuel avec un os long.

4. Dispositif (1) selon l'une des revendications précédentes, **caractérisé en ce que** la traverse (2) présente au moins deux ouvertures (3) qui sont placées le long de l'axe longitudinal (L) de la traverse (2).

5. Dispositif (1) selon l'une des revendications précédentes, **caractérisé en ce que** la traverse (2) présente des ouvertures (3) pour l'engrènement d'un outil pour se plier et/ou faire une torsion de la zone pliable (5) de la traverse.

6. Dispositif (1) selon l'une des revendications 4 ou 5, **caractérisé en ce que** les ouvertures (3) sont placées le long de l'axe longitudinal (1) de la traverse (2) à hauteur respectivement d'une griffe (4).

7. Dispositif (1) selon l'une des revendications 4 à 6, **caractérisé en ce que** les ouvertures (3) sont placées entre les zones configurées pliables (5) de la traverse (2).

8. Dispositif (1) selon l'une des revendications précédentes, **caractérisé en ce qu'**une ouverture (3) est prévue à hauteur de chaque griffe (4) du dispositif (1).

9. Dispositif (1) selon l'une des revendications précédentes, **caractérisé en ce que** le dispositif (1) présente une multitude de griffes (4) et/ou une multitude d'ouvertures (3), cependant que la structure du dispositif (1) se répète le long de l'axe longitudinal (L) de la traverse (2) à intervalles réguliers.
